# EUROPÄISCHE PATENTSCHRIFT

(11) **EP 1 423 492 B1**
(45) Veröffentlichungstag und Bekanntmachung des Hinweises auf die Patenterteilung: **02.07.2008**
(21) Anmeldenummer: 02797874.1
(22) Anmeldetag: 28.08.2002
(51) Int. Cl.: C11C 3/14, C07C 67/333, C07C 69/587

(54) **VERFAHREN ZUR HERSTELLUNG VON KONJUGIERTEN FETTSÄUREESTERN**
METHOD FOR PRODUCING CONJUGATED FATTY ACID ESTERS
PROCEDE DE PRODUCTION D'ESTERS D'ACIDES GRAS CONJUGUES

(30) Priorität: 06.09.2001 DE 10143534
(43) Veröffentlichungstag der Anmeldung: 02.06.2004
(73) Patentinhaber: Cognis IP Management GmbH, 40589 Düsseldorf (DE)
(72) Erfinder: ZANDER, Lars, 41569 Rommerskirchen (DE); STRUBE, Albert, 41470 Neuss (DE); HEIDBREDER, Andreas, 42699 Solingen (DE); WESTFECHTEL, Alfred, 40724 Hilden (DE); GIEDE, Wolfgang, 40764 Langenfeld (DE)
(86) Internationale Anmeldenummer: PCT/EP2002/009584
(87) Internationale Veröffentlichungsnummer: WO 2003/022964

(56) Entgegenhaltungen:
- WO-A-00/09163
- DE-C- 19 718 245
- GB-A- 1 408 189
- NICHOLS P L JR ET AL: "Isomers of conjugated fatty acids. I. Alkali-isomerized linoleic acid" JOURNAL OF THE AMERICAN CHEMICAL SOCIETY, AMERICAN CHEMICAL SOCIETY, WASHINGTON, DC, US, Bd. 73, Nr. 1, 24. Januar 1951 (1951-01-24), Seiten 247-252, XP002130752 ISSN: 0002-7863

## Beschreibung

### Gebiet der Erfindung

Die Erfindung betrifft ein Verfahren zur Herstellung von konjugierten Fettsäureestern mit verbesserter Aufarbeitung des Reaktionsgemischs.

### Stand der Technik

Ungesättigte Fettsäuren stellen wichtige Bausteine natürlicher Fette da. Als einer der wichtigsten Vertreter ist hier die Linolsäure zu nennen, bei der es sich um eine C18-Säure mit zwei Doppelbindungen handelt. 1978 wurde von Forschem an der Universität von Wisconsin entdeckt, daß Isomere dieser Fettsäure, bei der die Doppelbindungen konjugiert vorliegen, besonders günstige Auswirkungen auf die Gesundheit haben. Diese isomeren Octadecadiensäuren werden in der wissenschaftlichen Literatur unter dem Begriff konjugierte Linolsäuren (Abkürzung: CLA) zusammengefaßt und haben in letzter Zeit zunehmend Beachtung gefunden. NUTRITION, VOL: 19/NR. 6 1995. Sie sind verantwortlich für eine Reduktion des Fettanteils im Gewebe und unterstützen den Muskelaufbau. Desweiteren sollen sie anticancerogen wirken und das Imunsystem anregen.

Konjugierte Linolsäuren finden sich als Bestandteile in verschiedenen Lebensmitteln. Ihre Hauptquelle sind die tierischen Lebensmittel, aber auch in Milch und Milchprodukten sind bedeutende CLA-Mengen enthalten. Des weiteren wurde in verschiedenen Ölen und Fetten CLA nachgewiesen, wobei die Konzentration in pflanzlichen Ölen bedeutend niedriger war als jene in tierischen Fetten. J.Food Compos. Anal. 5,185-197 (1992).

Da der Gehalt dieser Lebensmittel stark schwanken kann, ist man daran interessiert, Nahrungsmitteln synthetisch hergestellte konjugierte Linolsäure zuzusetzen. Die EP 0 950 410 A1 offenbart ein Verfahren zur Isomerisierung von Linolsäure in Form eines stark linolsäurehaltigen Alkylesters mit einer starken Base, vorzugsweise mit Alkalialkoholaten, in wasserfreiem Medium. Die Gewinnung der Alkylester erfolgt wie es im Buch von G. Dieckelmann und H.J. Heinz "The basics of industrial oleochemistry"(ISBN 3-89355-008-9) beschrieben ist GB-A-1 408 189 und WO-A-00/09163 offenbaren Verfahren zur Isomerisierung ungesättigtes Fettsäureester unter Verwendung von Alkalialkoholaten.

Die konjugierte Linolsäure oder der entsprechende Ester wird durch Neutralisation mittels Säure in wäßriger Lösung aufgearbeitet. Dabei hat sich gezeigt, daß bei Durchführung der Isomerisierung an Estern eine höhere Ausbeute an konjugierter Linolsäure erhalten wird. Daher wird die Isomerisierung meist am Methylester unter Verwendung von Alkalimethanolaten durchgeführt. Nach der Zugabe von Wasser und Säure kommt es zu einer Phasentrennung, bei der der Konjugierte Linolsäureester als organische Phase anfällt.

Bei dieser Aufarbeitung nach der Isomerisierung tritt jedoch meist das Problem auf, daß bei Zugabe von Wasser die bei der Reaktionsführung gebildeten Seifen eine eindeutige Phasentrennung verhindern, da eine Emulsion gebildet wird. Desweitem wird die wäßrige Phase durch das freigesetzte Methanol verunreinigt, welches aus umwelttechnischen Aspekten aufwendig entfernt werden muß.

Die Aufgabe der vorliegenden Erfindung hat nun darin bestanden, ein Verfahren zu entwickeln, das die Isolierung von konjugierten Alkylfettsäureestem nach der Isomerisierung vereinfacht. So soll verhindert werden, daß die Reaktionslösung stark schäumt oder Emulsionen bildet. Desweiteren soll der Einsatz von Lösungsmitteln beschränkt oder ganz vermieden werden, insbesondere sollen keine Wasser-Lösungsmittel-Gemische anfallen. Außerdem soll die Reinheit der Endprodukte erhöht werden, d.h. Produkte mit niedrigerer Säurezahl sollen erhalten werden.

### Beschreibung der Erfindung

Gegenstand der Erfindung ist ein Verfahren zur Herstellung von konjugierten ungesättigten Fettsäureestern, bei dem man
a) nicht konjugierte ungesättigte Fettsäureester mit 12 bis 22 C-Atomen und mindestens 2 Doppelbindungen im Fettsäureanteil und 1 bis 5 C-Atomen im Alkoholanteil mit starken wasserfreien Basen versetzt und dabei isomerisiert,
b) die dabei erhaltenen konjugierten Fettsäureester mit Phosphorsäure versetzt und
c) die dabei ausgefallenen Phosphatsalze vom Reaktionsgemisch abtrennt.

Auf diese Weise tritt nach der Isomerisierung keine Esterspaltung auf. Desweiteren kann wegen der Abwesenheit von Wasser und Seifen in diesem Verfahren kein Zweiphasensystem entstehen, dessen Aufarbeitung aufgrund von Emulsionsbildung, d.h. fehlender Phasentrennung, erschwert wird.

Vorzugsweise setzt man Fettsäureester mit 16 bis 18 C-Atomen und mindestens 2 Doppelbindungen im Fettsäureanteil und 1 bis 5 C-Atomen im Alkoholanteil ein. Besonders bevorzugt setzt man als Fettsäureester Linolsäureester mit 1 bis 5 C-Atomen im Alkoholanteil ein. Von diesen Fettsäureestern sind Linolsäuremethylester und Linolsäureethylester besonders bevorzugt. Auch linolsäurehaltige Öle, die ausgewählt sind aus der Gruppe, die gebildet wird von Sonnenblumenöl, Distelöl, Sojaöl, Baumwollsaatöl, Erdnußöl, Rüböl und Palmöl, können nach Überführung der freien Fettsäuren in die Ester mit Alkoholen mit 1 bis 5 C-Atomen als Ausgangsprodukt für die erfindungsgemäße Herstellung von konjugierten Fettsäureestern Verwendung finden.

In einer bevorzugten Ausführungsform werden Alkalialkoholate mit 1 bis 10 C-Atomen als Basen während der Isomerisierung verwendet. Bei Zusatz von Phosphorsäure fallen Alkaliphosphate aus der Reaktionslösung aus und können leicht abgetrennt werden. Der aus der Reaktion von Phosphorsäure mit Alkalialkoholaten anfallende Alkohol kann in einfacher Weise unter Vakuum abdestilliert werden. Somit entstehen beim Herstellungsprozeß keine kontaminierten Lösungsmittel, die teuer zu entsorgen sind.

Als besonders bevorzugte Basen haben sich Kaliummethanolat, Kaliumethanolat oder Kalium-t-butylat erwiesen. Das bei Verwendung dieser beiden Basen nach Zusatz von Phosphorsäure gebildete Kaliumphosphat besitzt die günstigsten Fällungseigenschaften und führt zu einer fast vollständigen Entfernung der Kaliumionen aus der Reaktionsmischung. Die nach Zusatz von Phosphorsäure gebildeten Alkohole Methanol, Ethanol oder t-Butanol lassen sich schnell und ohne starke Schaumbildung abdestillieren.

Bevorzugt setzt man 85 %ige Phosphorsäure bei der Fällung der Phosphatsalze ein, da auf diese Weise möglichst wenig Wasser in das Reaktionsgemisch eingebracht wird und die Phosphorsäure aufgrund ihrer hohen Viskosität noch handhabbar ist. Zur Begünstigung der Fällungsreaktion erhitzt man die Reaktionsmischung auf 40 bis 130 °C, bevorzugt auf 80 bis 120 °C und besonders bevorzugt auf 100 bis 120 °C.

In einem weiteren bevorzugten Verfahren versetzt man Fettsäureester mit 12 bis 22 C-Atomen und mit mindestens zwei nichtkonjugierten Doppelbindungen im Fettsäureanteil und 1 bis 5 C-Atomen im Alkoholanateil mit wasserfreien Basen, um die Fettsäureester zu isomerisieren, und tropft anschließend in die Reaktionslösung Glycerin ein. Die dabei erhaltenen konjugierten Fettsäuretriglyceride versetzt man mit Phosphorsäure und trennt die ausgefallenen Phosphatsalze vom Reaktionsgemisch ab.

### Beispiele

### Beispiel 1

### Herstellung von konjugiertem Sonnenblumenmethylester

1200g Sonnenblumenmethylester wurden vor der Konjugierung getrocknet und dann mit 24g Kaliummethanolat versetzt. Die Temperatur wurde auf 130°C erhöht. Danach wurde für 2,5-3 Stunden bei dieser Temperatur gerührt. Anschließend wurde der Ansatz auf 110°C abgekühlt und vorsichtig mit 85%iger Phosphorsäure neutralisiert, wobei sich die Lösung von rotbraun zu gelb verfärbte. Die Temperatur wurde auf 120°C erhöht und für 30 min im Vakuum bei 120°C gerührt. Anfänglich wurde ein leichtes Schäumen beobachtet, das nach kurzer Zeit verschwand. Das Produkt wurde auf 40°C abgekühlt und durch Filtration an Hyflow von den Kalium-Salzen befreit. Zusatz von 1% Ethoxyquin diente zur Stabilisierung. Der so erhaltene konjugierte Sonnenblumenmethylester wies eine Säurezahl von 8 auf (bestimmt nach ISO 660).

### Beispiel 2

### Herstellung von konjugiertem Sonnenblumenmethylester mit wäßriger Aufarbeitung

443 g Sonnenblumenmethylester wurden mit 10g 30%igem Kaliummethanolat versetzt. Die Mischung wurde 5 Stunden in einer geschlossenen Reaktionsapparatur bei 111 bis 115 °C gerührt. Die Reaktionslösung wurde auf 100 °C abgekühlt und mit 100 ml Wasser, dem 4 g Zitronensäure zugesetzt waren, versetzt.

Dabei schäumte die Reaktionslösung zuerst stark auf, danach bildete sich eine Emulsion aus, die keine vollständige Phasentrennung zuließ. Auf diese Weise konnte nur wenig konjugierter Sonnenblumenmethylester mit einer Säurezahl von 15 (bestimmt nach ISO 660) isoliert werden.

### Beispiel 3

### Herstellung von konjugierten Sonnenblumentriglyceriden

265g Sonnenblumenmethylester wurden vor der Konjugierung getrocknet und dann mit 5.3g Kaliummethanolat versetzt. Die Temperatur wurde auf 130°C erhöht. Danach wurde für 3 Stunden bei dieser Temperatur gerührt. In diese Lösung wurden 10g Glycerin eingetropft und das freiwerdende Methanol im Vakuum abdestilliert. Anschließend wurde der Ansatz auf 110°C abgekühlt und vorsichtig mit 85%iger Phosphorsäure neutralisiert, wobei sich die Lösung von rotbraun zu gelb verfärbte. Die Temperatur wurde auf 120°C erhöht und für 30 Minuten im Vakuum bei 120°C gerührt. Anfänglich wurde ein leichtes Schäumen beobachtet, das nach kurzer Zeit verschwand. Das Produkt wurde auf 40°C abgekühlt und durch Filtration an Hyflow von den Kalium-Salzen befreit. Zusatz von 1% Ethoxyquin diente zur Stabilisierung.

## Patentansprüche

1. Verfahren zur Herstellung von konjugierten ungesättigten Fettsäureestern, bei dem man
a) nicht konjugierte ungesättigte Fettsäureester mit 12 bis 22 C-Atomen und mindestens 2 Doppelbindungen im Fettsäureanteil und 1 bis 5 C-Atomen im Alkoholanteil mit wasserfreien Basen versetzt und dabei isomerisiert,
b) die dabei erhaltenen konjugierten Fettsäureester mit Phosphorsäure versetzt und
c) die dabei ausgefallenen Phosphatsalze vom Reaktionsgemisch abtrennt.

2. Verfahren nach Anspruch 1, **dadurch gekennzeichnet, daß** man als Fettsäureester Linolsäurealkylester einsetzt.

3. Verfahren nach Anspruch 1 oder 2, **dadurch gekennzeichnet, daß** man als Fettsäureester Linolsäuremethylester oder Linolsäureethylester einsetzt.

4. Verfahren nach den Ansprüchen 1 bis 3, **dadurch gekennzeichnet, daß** man als wasserfreie Basen Alkalialkoholate einsetzt.

5. Verfahren nach Anspruch 4, **dadurch gekennzeichnet, daß** man als wasserfreie Base Kaliummethanolat, Kaliumethanolat oder Kalium-t-butylat einsetzt.

6. Verfahren nach den Ansprüchen 1 bis 5, **dadurch gekennzeichnet, daß** man zum Ausfällen der Phosphorsalze eine 85 %ige Phosphorsäure einsetzt.

7. Verfahren nach den Ansprüchen 1-6, **dadurch gekennzeichnet, daß** man zwischen den Verfahrensschritten a) und b) Glycerin in die Reaktionslösung eintropft und den bei dieser Umesterung freiwerdenden Alkohol abdestilliert.

## Claims

1. A process for the production of conjugated unsaturated fatty acid esters, in which
a) water-free bases are added to unconjugated unsaturated fatty acid esters containing 12 to 22 carbon atoms and at least 2 double bonds in the fatty acid component and 1 to 5 carbon atoms in the alcohol component, so that the unconjugated unsaturated fatty acid esters are isomerized,
b) phosphoric acid is added to the conjugated fatty acid esters obtained and
c) the phosphate salts precipitated are removed from the reaction mixture.

2. A process as claimed in claim 1, **characterized in that** linoleic acid alkyl ester is used as the fatty acid ester.

3. A process as claimed in claim 1 or 2, **characterized in that** linoleic acid methyl ester or linoleic acid ethyl ester is used as the fatty acid ester.

4. A process as claimed in claims 1 to 3, **characterized in that** alkali metal alcoholates are used as the water-free bases.

5. A process as claimed in claim 4, **characterized in that** potassium methanolate, potassium ethanolate or potassium t-butylate is used as the water-free base.

6. A process as claimed in claims 1 to 5, **characterized in that** an 85% phosphoric acid is used to precipitate the phosphate salts.

7. A process as claimed in claims 1 to 6, **characterized in that** glycerol is added dropwise to the reaction solution between process steps a) and b) and the alcohol released during this transesterification is distilled off.

## Revendications

1. Procédé de préparation d'esters d'acides gras insaturés conjugués selon lequel :
a) on mélange des esters d'acides gras insaturés non conjugués ayant 12 à 22 atomes de carbone et au moins 2 doubles liaisons dans la partie acide gras et 1 à 5 atomes de carbone dans la partie alcool avec des bases anhydres et ainsi on les isomérise,
b) on mélange les esters d'acides gras conjugués ainsi obtenus avec de l'acide phosphorique, et
c) on sépare les sels phosphatés qui précipitent ainsi du mélange réactionnel.

2. Procédé selon la revendication 1,
**caractérisé en ce qu'**
en tant qu'ester d'acides gras, on met en oeuvre un alkyl ester d'acide linoléique.

3. Procédé selon la revendication 1 ou la revendication 2,
**caractérisé en ce qu'**
en tant qu'ester d'acides gras, on met en oeuvre du méthyl ester d'acide linoléique ou de l'éthyl ester d'acide linoléique.

4. Procédé selon les revendications 1 à 3,
**caractérisé en ce qu'**
en tant que bases anhydres, on met en oeuvre des alcoolates alcalins.

5. Procédé selon la revendication 4,
**caractérisé en ce qu'**
en tant que base anhydre, on met en oeuvre du formiate de potassium, de l'éthylate de potassium ou du t-butylate de potassium.

6. Procédé selon les revendications 1 à 5,
**caractérisé en ce que**
pour précipiter les sels phosphorés, on met en oeuvre un acide phosphorique à 85 %.

7. Procédé selon les revendications 1-6,
**caractérisé en ce qu'**
entre les étapes a) et b), on ajoute goutte à goutte du glycérol dans le mélange réactionnel et on élimine par distillation l'alcool libéré au cours de cette transestérification.
